# EUROPEAN PATENT SPECIFICATION

(11) **EP 2 044 807 B1**
(45) Date of publication and mention of the grant of the patent: **02.09.2009**
(21) Application number: 06762574.9
(22) Date of filing: 13.07.2006
(51) Int. Cl.: H04R 25/00, A61F 11/06

(54) **METHOD FOR IN-SITU MEASURING OF ACOUSTIC ATTENUATION AND SYSTEM THEREFOR**
VERFAHREN ZUR IN-SITU-MESSUNG DER AKUSTISCHEN DÄMPFUNG UND SYSTEM DAFÜR
PROCEDE DE MESURE IN SITU DE L'ATTENUATION ACOUSTIQUE ET SYSTEME ASSOCIE

(43) Date of publication of application: 08.04.2009
(73) Proprietor: Phonak AG, 8712 Stäfa (CH)
(72) Inventor: HARSCH, Samuel, CH-1338 Ballaigues (CH); VON DACH, Thomas, CH-1785 Cressier (CH)
(74) Representative: Schwan - Schwan - Schorer
(86) International application number: PCT/EP2006/006882
(87) International publication number: WO 2008/006393

(56) References cited:
- WO-A-20/05055656
- GB-A- 2 261 950
- US-A- 3 968 334
- US-A- 4 060 701
- US-A1- 2004 086 138

## Description

The present invention relates to a method and a system for measuring in-situ the acoustic attenuation provided by a hearing protection device worn by a user.

US 6,687,377 B2 relates to a method for measuring the acoustic attenuation provided by a customized earplug, wherein the earplug is provided with a sound bore extending from the outer side of the earplug to the inner end of the earplug, wherein a remote device is inserted into the outer end of the sound bore, which remote device comprises a first microphone oriented towards the sound bore and a second microphone oriented towards ambience, and wherein test sound is provided by an external loudspeaker. Both the loudspeaker and the remote device are connected to a computer unit on which a measurement program is run. The acoustic attenuation provided by the earplug is calculated from the sound level difference between the first microphone and the second microphone.

US 5,577,511 relates to a method for measuring in-situ the acoustic attenuation provided by an earplug, wherein a probe tube extends through the earplug into the ear canal and wherein the outer end of the probe tube is connected to a first microphone, while a second microphone is provided at the ear for measuring sound pressure levels exterior to the ear canal as a reference microphone. The acoustic attenuation provided by the earplug is calculated from the difference of the sound levels measured by the first and the second microphone. The test sound for the measurement is the user's voice.

US 6,567,524 B1 relates to a customized earplug which comprises an inner microphone oriented towards the ear canal, an outer microphone oriented towards ambience and a loudspeaker oriented towards the ear canal. The earplug may be used for online control and verification of the acoustic attenuation provided by the earplug by generating a test sound by the loudspeaker and analyzing the signal picked up by the inner microphone.

DE 101 17 705 A1 relates to a hearing protection headphone which comprises a microphone oriented towards ambience and into which a hearing protection earplug is integrated which comprises a loudspeaker oriented towards the ear canal and a microphone oriented towards the ear canal for picking up sound in the ear canal for active noise reduction purposes. The microphone of the headphone is used for selectively providing ambient sound to the ear canal via the loudspeaker of the earplug for communication purposes.

It is an object of the invention to provide for a reliable and reproducible method for measuring in-situ the acoustic attenuation provided by a hearing protection device. It is a further object to provide for a corresponding system.

These objects are achieved by a method as defined in claim 1 and a system as defined in claim 11, respectively.

The invention is beneficial in that, by providing the hearing protection device with a microphone oriented towards the ear canal and by using a separate cup-like measuring device which creates a closed space surrounding the hearing protection device and which comprises a loudspeaker for providing a test sound to the closed space and a microphone for picking up the level of the test sound in the closed space, particularly reliable and reproducible in-situ measurements of the acoustic attenuation provided by the hearing protection device can be obtained, since well-defined test conditions are provided by the arrangement of the hearing protection device and the measuring device. In addition, by utilizing this concept, the modifications to the hearing protection device required for the in-situ measurement with regard to a standard hearing protection device can be kept low.

Preferred embodiments of the invention are described in the dependent claims.

In the following, examples of the invention will be illustrated by reference to the attached drawings, wherein:
- Fig. 1: shows schematically an example of an in-situ acoustic attenuation measurement system according to the invention;
- Fig. 2: Fig. 2 is a more detailed schematic view of one ear of the user during an in-situ acoustic attenuation measurement;
- Fig. 3: is a schematic side view, partially in cross-section, of a hearing protection earplug to be used in the invention;
- Fig. 4: is a top view of a holder used for calibrating the microphones used in an in-situ acoustic attenuation measurement system according to the invention; and
- Fig. 5: is a view of a headphone and the holder of Fig. 4 during a calibration measurement according to the invention.

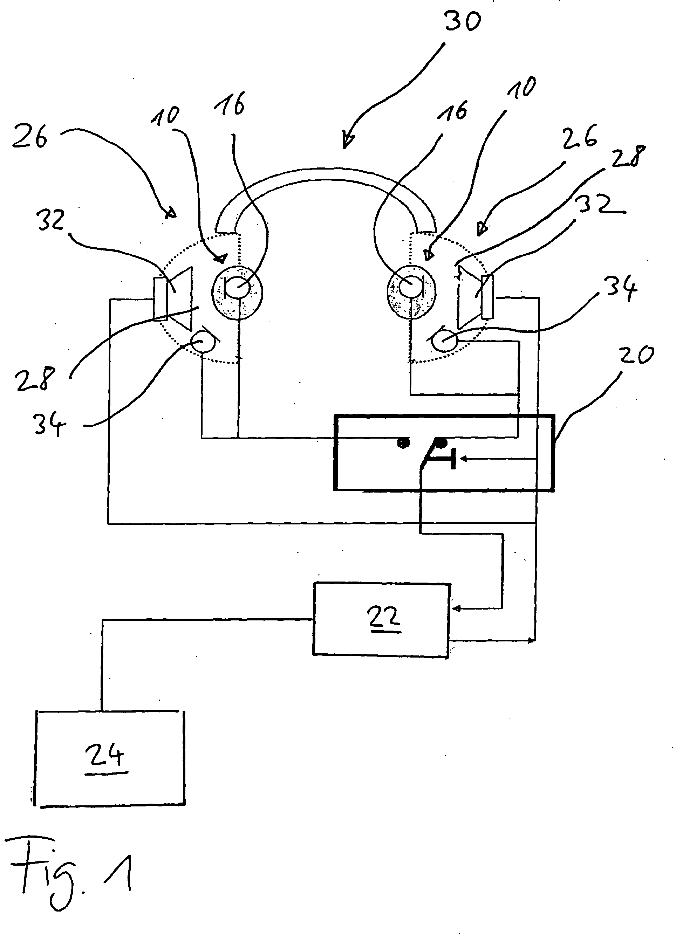

Fig. 1 is a schematic view of a system for measuring the acoustic attenuation obtained by hearing protection device 10 worn by a user. Preferably, the hearing protection device 10 is a customized earplug 10 comprising a hard shell 12 which may have an elasticity from shore D85 to D65 and may be made of polyamide. The earplug 10 is inserted at least in part into the user's ear canal 14 in an attenuation position. In order to achieve optimized fit of the shell 12 within the user's out ear and ear canal, the shell 12 has an outer surface individually shaped according to the measured shape of the user's outer ear and ear canal. The shape of the user's outer ear and ear canal may be determined by direct three-dimensional scanning of the ear canal of the concha or by producing an impression of the ear canal and the concha which subsequently undergoes scanning. The digital data obtained by the scanning process is then used to create the hard shell by an additive or incremental layer-by-layer build-up process. Examples of such processes for producing customized earplugs can be found, for example, in US 2003/0133583 A1 or US 6,533,062 B1.

The earplug 10 includes a microphone 16 which is oriented towards the user's ear canal 14 and the user's ear drum 19 for capturing sound in the ear canal 14. The acoustic connection of the microphone 16 to the ear canal 14 usually will is obtained by providing a corresponding sound passage 18 in the earplug 10, which extends from the microphone 16 to the inner end of the earplug 10.

The microphone 16 is electrically connected to a means for processing the audio signals captured by the microphone 16 from the sound in the ear canal 14. According to the example shown in Fig. 1 the microphone 16 for this purpose may be connected via an automatic switch 20 and a sound card 22 to a computer 24, for example via a USB interface.

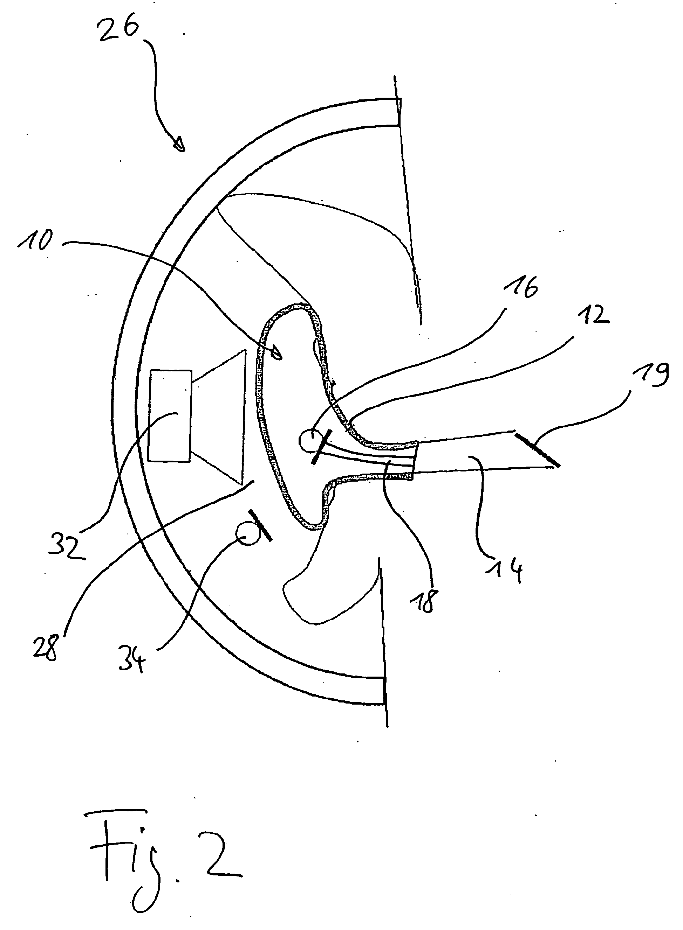

The system also comprises a cup-like measuring device 26 which is positioned at the user's ear in such a manner that a closed space 28 surrounding the earplug 10 positioned in the ear canal 14 is created by the measuring device 26. Preferably, as shown in Figs. 1 and 2, the measuring device 26 is one cup of a headphone 30. The measuring device 26 comprises a loudspeaker 32 which is oriented towards the user's ear for providing test sound to the closed space 28 and a microphone 34 which is oriented towards the user's ear for picking up sound in the closed space 28 in order to capture audio signals from the sound in the closed space 28. The microphone 34 is electrically connected via the automatic switch 20 and the sound card 22 to the computer 24, while the loudspeaker 32 is connected via the sound card 22 to the computer 24.

According to Fig. 1, the system is binaural, i.e. at each ear an earplug 10 and a measuring device 26 are provided. The switch 20 serves to switch between an acoustic attenuation measurement for the left ear and the right ear, with the measurement being performed subsequently for the two ears.

For carrying-out an in-situ acoustic attenuation measurement first an earplug 10 is inserted into the user's ear canal 14 of his left ear and right ear, respectively. Subsequently, the headphone 30 is positioned at the user's head in such a manner that the closed space 28 is formed within each cup 26 around each earplug 10. The microphones 16, the microphones 34 and the loudspeakers 28 are connected to the computer 24 via the sound card 22. Before the actual measurement starts, an audio signal is generated by the computer 24 and the sound card 22 which sets the switch 20, for example, to the right ear position as shown in Fig. 1. Then a test audio signal is generated by the computer 24 and the sound card 22 and is supplied to the loudspeaker 32 which creates a corresponding test sound in the closed space 28. This test sound is directly picked-up by the microphone 34 of the cup 26, and a corresponding audio signal is supplied via the sound card 22 to the computer 24. Simultaneously the test sound as attenuated by action of the earplug 10 is picked-up by the microphone 16 of the earplug 10 from the ear canal 14 which is acoustically sealed by the earplug 10, and a corresponding audio signal is supplied via the sound card 22 to the computer 24. The audio signals from the microphones 16 and 34 undergo some signal processing, such as recording, Fast Fourier Transformation (FFT) and conversion in octave bands, in the computer 24, and subsequently the acoustic attenuation provided by the earplug 10 can be estimated from the processed audio signals, i.e. from the sound level measured by each of the microphones 16 and 34.

Subsequently an audio signal is generated by the computer 24 and the sound card 22, which acts upon the automatic switch 20 in such a manner that it changes into the position for the left ear measurement. Then the attenuation measurement described above is performed for the left ear.

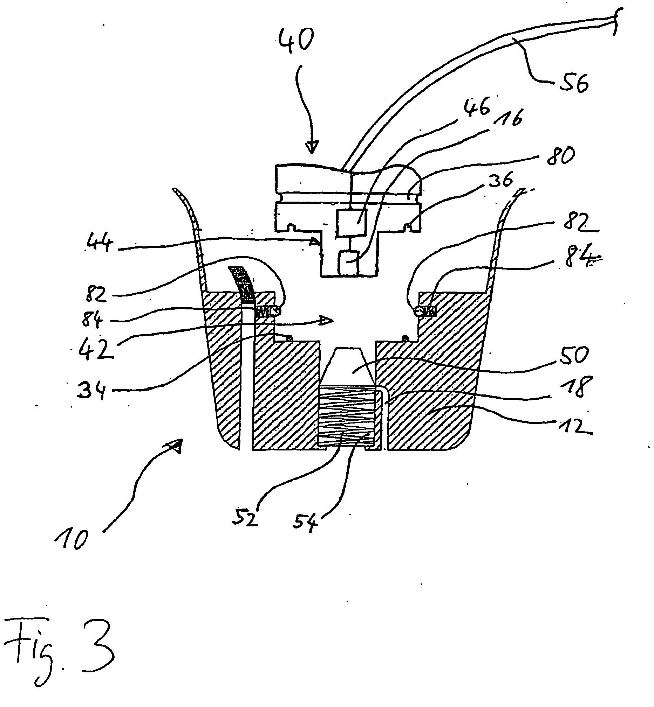

According to a preferred embodiment the earplug 10 is designed such that the microphone 16 is part of a measurement module 40 which can be detachably connected to the shell 12 for carrying out the attenuation measurement. According to Fig. 3 the shell 12 comprises an outer cavity 42 into which the measurement module 40 is to be inserted in a detachable manner. To this end, the shell 12 is provided with radially movable engagement elements 82 which are radially biased towards the centre of the shell 12 by spring elements 84. The engagement elements 82 may be balls. The measurement module 40 is provided with a circumferential groove 80 at the outer surface into which the engagement elements 82 may engage. Further, the cavity 42 is provided with a sealing lip 34 adapted for engagement with a mating groove 36 at the measurement module 40.

In addition, the shell 12 comprises an acoustic valve 48 at the inner end of the cavity 42, which is formed by a conical valve body 50 which is outwardly biased by a spring 52 in a recess 54 and which is tapered outwardly. In the closed position of the valve 48 shown in Fig. 3 the sound passage 18 extending from the cavity 42 to the inner end of the shell 12 is acoustically closed by the valve body 50. However, when the measurement module 40 is inserted into the cavity 42, the valve body 50 is forced inwardly against the biasing force of the spring 52 by an axial extension 44 of the measurement module 40 so that the sound passage 18 is opened for acoustic connection to the microphone 16 of the measurement module.

The measurement module 40 also may comprises an electronic unit 46 connected to the microphone 16 for performing signal processing of the audio signals provided by the microphone 16, for example, pre-amplification. Finally, the measurement module 40 comprises an electrical connection 56 to the automatic switch 20.

When the acoustic attenuation measurement has been terminated, the measurement module 40 may be removed again from the earplug 10 and may be replaced by a functional module (not shown) which is connected to the shell 12 in a detachable manner similar to the measurement module 40. Such functional module may include an acoustic filter and/or a wireless communication unit for the actual use of the earplug 10 by the user.

The mechanism for connecting the measurement module 40 to the earplug 10 in a detachable manner may be any appropriate quickly detachable connection, such as a clipping connection, a screwing connection or a bayonet coupling. Examples of such connections are given in EP 1 674 059 A1.

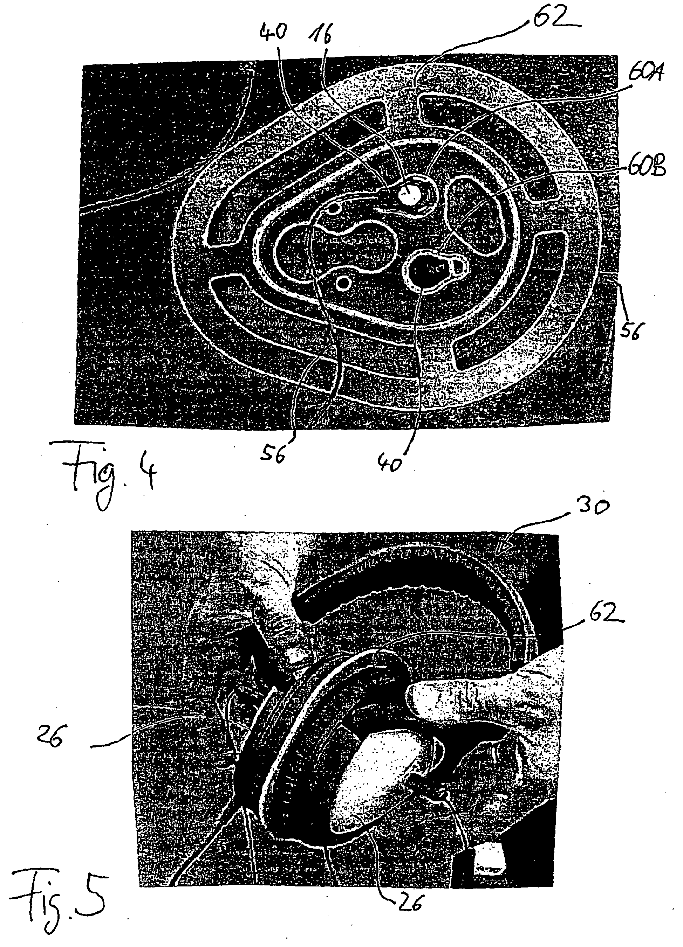

Preferably, the microphone of the hearing protection device and the microphone of the measuring device are calibrated relative to each other prior to the in-situ attenuation measurement. For example, for a measurement device 26 of the type shown in Fig. 1 and an earplug 10 of the type shown Fig. 3 such calibration can be effected by the procedure shown in Figs. 4 and 5.

In a first step, the measurement modules 40 with the microphone 16 for the left ear earplug 10 and the right ear earplug 10 are mounted in a mating receptacle 60A and 60B, respectively, of a plate-like holder 62, preferably in such a manner that the microphones 16 are facing opposite sides. Each measurement module 40 is connected via the electrical connection 56 in the manner of Fig. 1 via the switch 20 to the sound card 22 and thus to the computer 24.

In a second step, the holder 62 with the measurement modules 40 mounted on it is placed between the cups 26 of the measurement headphone 30. The cups 26 are manually pressed together so that the holder 62 is sandwiched in-between in a manner that each cup 26 creates a closed space around the respective measurement module 40, i.e. the respective microphone 16.

Before the actual calibration measurement starts, an audio signal is generated by the computer 24 and the sound card 22 which sets the switch 20, for example, to the right ear position. Then a test audio signal is generated by the computer 24 and the sound card 22 and is supplied to the loudspeaker 32 which creates a corresponding test sound in the closed space 28. This test sound is directly picked-up by the microphone 34 of the right ear cup 26, and a corresponding audio signal is supplied via the sound card 22 to the computer 24. Simultaneously the test sound is also directly picked-up by the microphone 16 of the measurement module 40 mounted in the receptacle 60A, and a corresponding audio signal is supplied via the sound card 22 to the computer 24. The audio signals from the microphones 16 and 34 undergo some signal processing in the computer 24, and subsequently one calibration factor or a set of calibration factors applicable for the microphone 34 of the right ear cup 26 relative to the microphone 16 of the right ear measurement module 40 is determined from the processed audio signals, i.e. from the audio signal level provided by each of the microphones 16 and 34. This calibration factor or set of calibration factors is then used as correction for the audio signal levels obtained in the above described right ear attenuation measurement. In order to be sure that the correct one of the two measurement modules 40 is used in the right ear attenuation measurement, the measurement modules 40 are marked in order to be easily distinguished, for example by a colour code.

Subsequently an audio signal is generated by the computer 24 and the sound card 22, which acts upon the automatic switch 20 in such a manner that it changes into the position for the left ear measurement. Then the calibration measurement described above is performed for the left ear cup 26 and the microphone 16 of the measurement module 40 mounted in the receptacle 60B in order to obtain the calibration factor or set of calibration factors for the microphone 34 of the left ear cup 26 and the microphone 16 of the left ear measurement module 40.

## Claims

1. A method for measuring the acoustic attenuation obtained by a hearing protection device (10) worn by a user, comprising:
positioning the hearing protection device (10) at the user's ear in an attenuation position, said hearing protection device (10) comprising a microphone (16) oriented towards the user's ear canal (14) for capturing sound in the user's ear canal (14);
positioning a cup-like measuring device (26) at the user's ear in such a manner that a closed space (28) surrounding the hearing protection device positioned in the attenuation position is created by the measuring device (26), said measuring device (26) comprising a loudspeaker (32) oriented towards to the user's ear for providing sound to said closed space (28) and a microphone (34) oriented towards the user's ear for capturing audio signals from sound in said closed space (28);
providing a test signal via the loudspeaker (32), capturing audio signals representative of the level of the test signal in the closed space (28) by the microphone (34) of the measuring device (26) and capturing audio signals representative of the level of the test signal in the user's ear canal (14) by the microphone (16) of the hearing protection device (10);
processing the audio signals captured by the microphone (34) of the measuring device (26) and the audio signals captured by the microphone (16) of the hearing protection device (10) and estimating the attenuation provided by the hearing protection device (10) from the processed audio signals.

2. The method of claim 1, wherein the processing of the audio signals captured by the microphone (34) of the measuring device (26) and of the audio signals captured by the microphone (16) of the hearing protection device (10) includes Fast Fourier Transformation and conversion in octave bands.

3. The method of one of claims 1 and 2, wherein the hearing protection device (10) and the measuring device (26) are connected via an interface to an external device (22, 24) which generates said test signal and processes the audio signals captured by the microphone (34) of the measuring device (26) and the audio signals captured by the microphone of the hearing protection device (10) and estimates the attenuation provided by the hearing protection device (10).

4. The method of one of the preceding claims, wherein the hearing protection device is an earplug (10) which is inserted at least in part into the user's ear canal (14), and wherein the earplug (10) comprises a sound passage (18) extending from the microphone (16) to that end of the earplug (10) facing the user's ear drum (19).

5. The method of claim 4, wherein the microphone (16) of the earplug (10) is part of a measurement module (40) which is connected to the earplug (10) in a detachable manner prior to the acoustic attenuation measurement and wherein the measurement module (40) is removed from the earplug (10) after the acoustic attenuation measurement.

6. The method of claim 5, wherein after the acoustic attenuation measurement the measurement module (40) is replaced by a functional module which is connected to the earplug (10) in a detachable manner.

7. The method of claim 6, wherein the functional module includes an acoustic filter and/or a wireless communication unit.

8. The method of one of the preceding claims, wherein the method is subsequently applied to both ears of the user.

9. The method of one of the preceding claims, wherein prior to measuring the acoustic attenuation the microphone (16) of the hearing protection device (10) and the microphone (34) of the measuring device (26) are calibrated relative to each other by:
mounting the microphone (16) of the hearing protection device (10) at a holder (62);
positioning the measuring device (26) at the holder (62) in such a manner that a closed space (28) surrounding the microphone (16) of the hearing protection device (10) mounted at the holder (62) is created by the measuring device (26);
providing a test signal via the loudspeaker (32), capturing audio signals representative of the level of the test signal in the closed space (28) by the microphone (34) of the measuring device (26) and capturing audio signals representative of the level of the test signal in the closed space by the microphone (16) of the hearing protection device (10); and
comparing the audio signals captured by the microphone (16) of the hearing protection device (10) and the audio signal captured by the microphone (34) of the measuring device (36).

10. The method of claim 9, wherein the microphone (16) of the hearing protection device (10) is mounted at the holder (62) separate from the hearing protection device (10) for the calibration measurement and is detachably mounted at the hearing protection device (10) after the calibration measurement has been terminated, wherein the holder (62) has a plate-like shape and wherein the cup-like part of the measuring device is a cup (26) of a headphone (30), which is pressed against the holder (62) for creating said enclosed space (28), and wherein the holder (62) is placed between the two cups (26) of the headphone (30) which are pressed together.

11. A system for measuring the acoustic attenuation obtained by a hearing protection device (10) worn by a user, comprising:
a hearing protection device (10) capably of being positioned at the user's ear in an attenuation position and comprising a microphone (16) oriented towards the user's ear canal (14), when said hearing protection device is positioned in alternation position, for capturing audio signals from sound in the user's ear canal (14);
a cup-like measuring device (26) capable of being positioned at the user's ear in such a manner that a closed space (28) surrounding the hearing protection device (10) positioned in the attenuation position is created by the measuring device (26), said measuring device comprising (26) a loudspeaker (32) oriented towards the user's ear for providing sound to said closed space, (28) and a microphone (34) oriented towards the user's ear for capturing audio signals from sound in said closed space (28);
means for providing a test signal via the loudspeaker (32) to said closed space (28); and
means (22, 24, 46) adapted for processing the audio signals captured by the microphone (34) of the measuring device (26) and the audio signals captured by the microphones (16) of the hearing protection device (10) and adapted for estimating the attenuation provided by the hearing protection device (10) from the processed audio signals.

12. The system of claim 11, wherein the hearing protection device (10) and the measuring device (26) comprise an interface (56) for being connected to an external device (22, 24) adapted for generating said test signal and for processing the audio signals captured by the microphone (34) of the measuring device (26) and the audio signals captured by the microphone (16) of the hearing protection device (10) and estimating the attenuation provided by the hearing protection device (10).

13. The system of one of the claims 11, and 12, wherein the hearing protection device is an earplug (10) comprising a shell (12) to be inserted into the user's ear canal (14), and wherein the shell (12) comprises a sound passage (18) extending from the microphone (16) to that end of the earplug (10) which is to face the user's ear drum (19).

14. The system of claim 13, wherein the microphone (16) of the earplug (10) is part of a measurement module (40) which is detachably connected to the shell (12), and wherein the shell (12) comprises valve means (48, 50, 52) which are moveable, upon connecting the measurement module (40) to the shell (12), from a closed position in which the valve means (48, 50, 52) acoustically closes the sound passage (18) into an open position in which the valve means (48, 50, 52) acoustically opens the sound passage (18), the valve means (48, 50, 52) being biased towards the closed position in order to acoustically close the sound passage (18) when the measurement module (40) is removed from the shell (12), and wherein the microphone (16) is acoustically connected to the sound passage (18) when said measurement module (40) is connected to the shell (12).

15. The system of one of claims 11 to 14, wherein the cup-like part of the measuring device is a cup (26) of a headphone (30).

## Patentansprüche

1. Verfahren zum Messen der mittels einer von einem Anwender getragenen Gehörschutzvorrichtung (10) erzielten akustischen Dämpfung, wobei:
die Gehörschutzvorrichtung (10) am Ohr des Anwenders in einer Dämpfungsposition positioniert wird, wobei die Gehörschutzvorrichtung (10) ein zum Gehörgang (14) des Anwenders hin orientiertes Mikrofon (16) zum Auffangen von Schall im Gehörgang (14) des Anwenders aufweist;
eine becherartige Meßvorrichtung (26) am Ohr des Anwenders in einer solchen Weise positioniert wird, dass mittels des Messgeräts (26) ein umschlossener Raum (28) erzeugt wird, welcher die in der Dämpfungsposition angeordnete Gehörschutzvorrichtung umgibt, wobei das Messgerät (26) einen zum Ohr des Anwenders hin orientierten Lautsprecher (32) zur Schallabgabe in den umschlossenen Raum (28) sowie ein zum Ohr des Anwenders hin orientiertes Mikrofon zum Auffangen von Audiosignalen aus dem Schall in dem umschlossenen Raum (28) aufweist;
ein Testsignal über den Lautsprecher (32) abgegeben wird, mittels des Mikrofons (34) der Meßvorrichtung (26) Audiosignale aufgefangen werden, die für den Pegel des Testsignals in dem umschlossenen Raum (28) repräsentativ sind, sowie mittels des Mikrofons (16) der Gehörschutzvorrichtung (10) Audiosignale aufgefangen werden, die für den Pegel des Testsignals im Gehörgang (14) des Anwenders repräsentativ sind;
die mittels des Mikrofons (34) der Meßvorrichtung (26) aufgefangenen Audiosignale und die mittels des Mikrofons (16) der Gehörschutzvorrichtung (10) aufgefangenen Audiosignale verarbeitet und aus den verarbeiteten Audiosignalen die durch die Gehörschutzvorrichtung (10) geleistete Dämpfung abgeschätzt wird.

2. Verfahren gemäß Anspruch 1, wobei das Verarbeiten der mittels des Mikrofons (34) der Meßvorrichtung (26) aufgefangenen Audiosignale und der mittels des Mikrofons (16) der Gehörschutzvorrichtung (10) aufgefangenen Audiosignale Schnelle-FourierTransformation und Umwandlung in Oktavbänder beinhaltet.

3. Verfahren gemäß einem der Ansprüche 1 und 2, wobei die Gehörschutzvorrichtung (10) und die Meßvorrichtung (26) mittels einer Schnittstelle mit einer externen Vorrichtung (22, 24) verbunden sind, welche das Testsignal erzeugt und die mittels des Mikrofons (34) der Meßvorrichtung (26) aufgefangenen Audiosignale und die mittels des Mikrofons der Gehörschutzvorrichtung (10) aufgefangenen Audiosignale verarbeitet sowie die von der Gehörschutzvorrichtung (10) geleistete Dämpfung abschätzt.

4. Verfahren gemäß einem der vorhergehenden Ansprüche, wobei es sich bei der Gehörschutzvorrichtung um einen Ohrstopfen (10) handelt, welcher mindestens zum Teil in den Gehörgang (14) des Anwenders eingeführt ist, und wobei der Ohrstopfen einen Schalldurchlass (18) aufweist, der sich von dem Mikrofon (16) zu dem Ende des Ohrstopfens (10) erstreckt, welches dem Trommelfell (19) des Anwenders zugewandt ist.

5. Verfahren gemäß Anspruch 4, wobei das Mikrofon (16) des Ohrstopfens (10) einen Teil eines Meßmoduls (40) bildet, welches vor der akustischen Dämpfungsmessung mit dem Ohrstopfen (10) lösbar verbunden wird, und wobei das Meßmodul nach der akustischen Dämpfungsmessung von dem Ohrstopfen (10) entfernt wird.

6. Verfahren gemäß Anspruch 5, wobei das Meßmodul (40) nach der akustischen Dämpfungsmessung durch ein funktionales Modul ersetzt wird, welches mit dem Ohrstopfen (10) in lösbarer Weise verbunden wird.

7. Verfahren gemäß Anspruch 6, wobei das funktionale Modul ein akustisches Filter und/oder eine drahtlose Kommunikationseinheit aufweist.

8. Verfahren gemäß einem der vorhergehenden Ansprüche, wobei das Verfahren nacheinander auf beide Ohren des Anwenders angewandt wird.

9. Verfahren gemäß einem der vorhergehenden Ansprüche, wobei vor dem Messen der akustischen Dämpfung das Mikrofon (16) der Gehörschutzvorrichtung (10) und das Mikrofon (34) der Meßvorrichtung (26) relativ zueinander kalibriert werden, indem:
das Mikrofon (16) der Gehörschutzvorrichtung (10) an einem Halter (62) montiert wird;
die Meßvorrichtung (26) dergestalt an dem Halter (62) positioniert wird, dass mittels der Meßvorrichtung (26) ein umschlossener Raum (28) erzeugt wird, der das Mikrofon (16) der an dem Halter (62) montierten Gehörschutzvornchtung (10) umgibt;
ein Testsignal über den Lautsprecher (32) abgegeben wird, mittels des Mikrofons (34) der Meßvorrichtung (26) Audiosignale aufgefangen werden, die für den Pegel des Testsignals in dem umschlossenen Raum (28) repräsentativ sind, und mittels des Mikrofons (16) der Gehörschutzvorrichtung (10) Audiosignale aufgefangen werden, die für den Pegel des Testsignals in dem umschlossenen Raum repräsentativ sind; und
die mittels des Mikrofons (16) der Gehörschutzvorrichtung (10) aufgefangenen Audiosignale und die mittels des Mikrofons (34) der Meßvorrichtung (26) aufgefangenen Audiosignale verglichen werden.

10. Verfahren gemäß Anspruch 9, wobei das Mikrofon (16) der Gehörschutzvorrichtung (10) für die Kalibtierungsmessung getrennt von der Gehörschutzvorrichtung (10) am Halter (62) montiert wird und nach Beendigung der Kalibrierungsmessung lösbar an der Gehörschutzvorrichtung (10) montiert wird, wobei der Halter (62) plattenförmig ausgebildet ist und wobei es sich bei dem becherförmigen Teil der Meßvorrichtung um eine Muschel (26) eines Kopfhörers (30) handelt, die gegen den Halter (62) gedrückt wird, um den umschlossenen Raum (28) zu erzeugen, und wobei der Halter (62) zwischen die beiden Muscheln (26) des Kopfhörers (30) platziert wird, die zusammengepresst werden.

11. System zum Messen der mittels einer von einem Anwender getragenen Gehörschutzvorrichtung erzielten akustischen Dämpfung, mit:
einer Gehörschutzvorrichtung (10), die am Ohr des Anwenders in einer Dämpfungsposition positioniert werden kann und ein zum Gehörgang (14) des Anwenders hin orientiertes Mikrofon (16) zum Auffangen von Audiosignalen aus Schall im Gehörgang (14) des Anwenders, wenn die Gehörschutzvorrichtung in der Dämpfungsposition positioniert ist, aufweist;
einer becherartigen Meßvorrichtung (26), die am Ohr des Anwenders derart positioniert werden kann, dass ein umschlossener Raum (28) mittels der Meßvorrichtung (26) erzeugt wird, welcher die in der Dämpfungsposition positionierte Gehörschutzvorrichtung (10) umgibt, wobei die Meßvorrichtung (26) einen Lautsprecher (32), der zum Ohr des Anwenders hin orientiert ist, um Schall in den umschlossenen Raum (28) abzugeben, sowie ein Mikrofon (34) aufweist, das zum Ohr des Anwenders hin orientiert ist, um Audiosignale aus Schall in dem umschlossenen Raum (28) aufzufangen;
Mitteln, um ein Testsignal über den Lautsprecher (32) in den umschlossene Raum (28) abzugeben; sowie
Mitteln (22, 24, 46) zum Verarbeiten der mittels des Mikrofons (34) der Meßvorrichtung (26) aufgefangenen Audiosignale und der mittels des Mikrofons (16) der Gehörschutzvorrichtung (10) aufgefangenen Audiosignale sowie zum Abschätzen der von der Gehürschutzvorrichtung (10) bewirkten Dämpfung aus den verarbeiteten Audiosignalen.

12. System gemäß Anspruch 11, wobei die Gehörschutzvorrichtung (10) und die Meßvorrichtung (26) eine Schnittstelle (56) zwecks Verbindung mit einer externen Vorrichtung (22, 24) aufweisen, die zum Erzeugen des Testsignals und zum Verarbeiten der mittels des Mikrofons (34) der Meßvorrichtung (26) aufgefangenen Audiosignale und der mittels des Mikrofons (16) der Gehörschutzvorrichtung (10) aufgegangenen Audiosignale sowie zum Abschätzen der von der Gehörschutzvorrichtung (10) bewirkten Dämpfung ausgebildet ist.

13. System gemäß einem der Ansprüche 10 und 11, wobei es sich bei der Gehörschutzvorrichtung um einen Ohrstopfen (10) mit einer in den Gehörgang (14) des Anwenders (1) einzuführenden Schale (12) handelt und wobei die Schale (12) einen Schalldurchlass (18) aufweist, der sich von dem Mikrofon (16) zu dem Ende des Ohrstopfens (10) erstreckt, welches dem Trommelfell (19) des Anwenders zuzuwenden ist.

14. System gemäß Anspruch 13, wobei das Mikrofon (16) des Ohrstopfens (10) ein Teil eines Meßmoduls (40) ist, welches lösbar mit der Schale (12) verbunden ist, und wobei die Schale (12) eine Ventilanordnung (48, 50, 52) aufweist, die, nach dem Verbinden des Meßmoduls mit der Schale (12), von einer geschlossenen Position, in welcher die Ventilanordnung (48, 50, 52) den Schalldurchlass (18) akustisch schließt, in eine offene Position bewegbar ist, in welcher die Ventilanordnung (48, 50, 52) den Schalldurchlass akustisch freigibt, wobei die Ventilanordnung (48, 50, 52) zu der geschlossenen Position hin vorgespannt ist, um den Schalldurchlass (18) akustisch zu schließen, wenn das Meßmodul (40) von der Schale (12) entfernt wird, und wobei das Mikrofon (16) mit dem Schalldurchlass (18) akustisch verbunden ist, wenn das Meßmodul (40) mit der Schale (12) verbunden ist.

15. System gemäß einem der Ansprüche 11 bis 14, wobei es sich bei dem becherförmige Teil der Meßvorrichtung um eine Muschel (26) eines Kopfhörers (30) handelt.

## Revendications

1. Procédé de mesure de l'atténuation acoustique fournie par un dispositif de protection auditive (10) porté par un utilisateur, comprenant les étapes consistant à :
placer le dispositif de protection auditive (10) dans l'oreille de l'utilisateur dans une position d'atténuation, ledit dispositif de protection auditive (10) comprenant un microphone (16) orienté vers le canal auriculaire de l'utilisateur (14) afin de capturer le son dans le canal auriculaire de l'utilisateur (14) ;
placer un dispositif de mesure (26) en forme de coupelle sur l'oreille de l'utilisateur, de manière à ce qu'un espace clos (28) entourant le dispositif de protection auditive placé dans la position d'atténuation soit créé par le dispositif de mesure (26), ledit dispositif de mesure (26) comprenant un haut-parleur (32) orienté vers l'oreille de l'utilisateur afin de fournir du son dans ledit espace clos (28) et un microphone (34) orienté vers l'oreille de l'utilisateur pour capturer des signaux audio à partir du son présent dans ledit espace clos (28) ;
fournir un signal d'essai via le haut-parleur (32), capturer des signaux audio représentatifs du niveau du signal d'essai dans l'espace clos (28) à l'aide du microphone (34) du dispositif de mesure (26) et capturer des signaux audio représentatifs du niveau du signal d'essai dans le canal auriculaire de l'utilisateur (14) à l'aide du microphone (16) du dispositif de protection auditive (10) ;
traiter les signaux audio capturés par le microphone (34) du dispositif de mesure (26) et les signaux audio capturés par le microphone (16) du dispositif de protection auditive (10) et estimer l'atténuation fournie par le dispositif de protection auditive (10) à partir des signaux audio traités.

2. Procédé selon la revendication 1, dans lequel le traitement des signaux audio capturés par le microphone (34) du dispositif de mesure (26) et des signaux audio capturés par le microphone (16) du dispositif de protection auditive (10) comprend une transformation de Fourier rapide et une conversion en bandes d'octaves.

3. Procédé selon l'une des revendications 1 et 2, dans lequel le dispositif de protection auditive (10) et le dispositif de mesure (26) sont connectés par une interface à un dispositif extérieur (22, 24) qui produit ledit signal d'essai, traite les signaux audio capturés par le microphone (34) du dispositif de mesure (26) et les signaux audio capturés par le microphone (16) du dispositif de protection auditive (10) et estime l'atténuation fournie par le dispositif de protection auditive (10).

4. Procédé selon l'une des revendications précédentes, dans lequel le dispositif de protection auditive est un bouchon auriculaire (10) qui est inséré au moins partiellement dans le canal auriculaire de l'utilisateur (14) et dans lequel le bouchon auriculaire (10) comprend un passage sonore (18) qui s'étend entre le microphone (16) et l'extrémité du bouchon auriculaire (10) qui fait face au tympan (19) dans l'oreille de l'utilisateur.

5. Procédé selon la revendication 4, dans lequel le microphone (16) du bouchon auriculaire (10) fait partie d'un module de mesure (40) qui est connecté au bouchon auriculaire (10) de manière détachable avant la mesure de l'atténuation acoustique et dans lequel le module de mesure (40) est détaché du bouchon auriculaire (10) après la mesure de l'atténuation acoustique.

6. Procédé selon la revendication 5, dans lequel, après la mesure de l'atténuation acoustique, le module de mesure (40) est remplacé par un module fonctionnel qui est connecté au bouchon auriculaire (10) de manière détachable.

7. Procédé selon la revendication 6, dans lequel le module fonctionnel comprend un filtre acoustique et/ou une unité de communication sans fil.

8. Procédé selon l'une des revendications précédentes, dans lequel le procédé est appliqué successivement aux deux oreilles de l'utilisateur.

9. Procédé selon l'une des revendications précédentes, dans lequel, avant de mesurer l'atténuation acoustique, le microphone (16) du dispositif de protection auditive (10) et le microphone (34) du dispositif de mesure (26) sont étalonnés l'un par rapport à l'autre en procédant comme suit :
monter le microphone (16) du dispositif de protection auditive (10) sur un support (62) ;
placer le dispositif de mesure (26) sur le support (62) de manière à ce qu'un espace clos (28) entourant le microphone (16) du dispositif de protection auditive (10) monté sur le support (62) soit créé par le dispositif de mesure (26) ;
fournir un signal d'essai via le haut-parleur (32), capturer des signaux audio représentatifs du niveau du signal d'essai dans l'espace clos (28) à l'aide du microphone (34) du dispositif de mesure (26) et capturer des signaux audio représentatifs du niveau du signal d'essai dans l'espace clos à l'aide du microphone (16) du dispositif de protection auditive (10) ; et
comparer les signaux audio capturés par le microphone (16) du dispositif de protection auditive (10) et les signaux audio capturés par le microphone (34) du dispositif de mesure (26).

10. Procédé selon la revendication 9, dans lequel, pour la mesure d'étalonnage, le microphone (16) du dispositif de protection auditive (10) est monté sur le support (62) séparément du dispositif de protection auditive (10) et est monté de manière détachable sur le dispositif de protection auditive (10) après que la mesure d'étalonnage a été achevée, dans lequel le support (62) a une forme de plateau et dans lequel la partie en forme de coupe du dispositif de mesure est une coque (26) d'un écouteur (30) qui est appliquée contre le support (62) pour créer ledit espace clos (28) et dans lequel le support (62) est placé entre les deux coques (26) de l'écouteur (30), qui sont pressées l'une contre l'autre.

11. Système de mesure de l'atténuation acoustique fournie par un dispositif de protection auditive (10) porté par un utilisateur, comprenant :
un dispositif de protection auditive (10) pouvant être placé dans l'oreille de l'utilisateur dans une position d'atténuation et comprenant un microphone (16) orienté vers le canal auriculaire de l'utilisateur (14), lorsque ledit dispositif de protection auditive est placé dans une position d'atténuation, afin de capturer des signaux audio à partir du son dans le canal auriculaire de l'utilisateur (14) ;
un dispositif de mesure (26) en forme de coupelle pouvant être placé sur l'oreille de l'utilisateur, de manière à ce qu'un espace clos (28) entourant le dispositif de protection auditive (10) placé dans la position d'atténuation soit créé par le dispositif de mesure (26), ledit dispositif de mesure (26) comprenant un haut-parleur (32) orienté vers l'oreille de l'utilisateur afin de fournir du son dans ledit espace clos (28) et un microphone (34) orienté vers l'oreille de l'utilisateur pour capturer des signaux audio à partir du son présent dans ledit espace clos (28) ;
un moyen destiné à fournir un signal d'essai dans ledit espace clos (28) via le haut-parleur (32) ; et
un moyen (22, 24, 46) conçu pour traiter les signaux audio capturés par le microphone (34) du dispositif de mesure (26) et les signaux audio capturés par le microphone (16) du dispositif de protection auditive (10) et pour estimer l'atténuation fournie par le dispositif de protection auditive (10) à partir des signaux audio traités.

12. Système selon la revendication 11, dans lequel le dispositif de protection auditive (10) et le dispositif de mesure (26) comprennent une interface (56) pour se connecter à un dispositif extérieur (22, 24) conçu pour produire ledit signal d'essai et pour traiter les signaux audio capturés par le microphone (34) du dispositif de mesure (26) et les signaux audio capturés par le microphone (16) du dispositif de protection auditive (10) et estimer l'atténuation fournie par le dispositif de protection auditive (10).

13. Système selon l'une des revendications 11 et 12, dans lequel le dispositif de protection auditive est un bouchon auriculaire (10) comprenant un embout (12) qui est inséré dans le canal auriculaire de l'utilisateur (14) et dans lequel l'embout (12) comprend un passage sonore (18) qui s'étend entre le microphone (16) et l'extrémité du bouchon auriculaire (10) qui fait face au tympan (19) dans l'oreille de l'utilisateur.

14. Système selon la revendication 13, dans lequel le microphone (16) du bouchon auriculaire (10) fait partie d'un module de mesure (40) qui est connecté à l'embout (12) de manière détachable et dans lequel l'embout (12) comprend un moyen de soupape (48, 50, 52) qui, après la connexion du module de mesure (40) à l'embout (12), peut se déplacer entre une position fermée dans laquelle le moyen de soupape (48, 50, 52) ferme acoustiquement le passage sonore (18) et une position ouverte dans laquelle le moyen de soupape (48, 50, 52) ouvre acoustiquement le passage sonore (18), le moyen de soupape (48, 50, 52) étant précontraint en direction de la position fermée afin de fermer acoustiquement le passage sonore (18) lorsque le module de mesure (40) est déconnecté de l'embout (12) et dans lequel le microphone (16) est connecté acoustiquement au passage sonore (18) lorsque ledit module de mesure (40) est connecté à l'embout (12).

15. Système selon l'une des revendications 11 à 14, dans lequel la partie en forme de coupelle du dispositif de mesure est une coque (26) d'écouteur (30).
